# FASCICULE DE BREVET EUROPEEN

(11) **EP 0 737 478 B1**
(45) Date de publication et mention de la délivrance du brevet: **30.05.2001**
(21) Numéro de dépôt: 96400502.9
(22) Date de dépôt: 11.03.1996
(51) Int. Cl.: A61K 33/22, A61K 33/14, A61K 33/06, A61K 33/00, A61K 31/19

(54) **Utilisation de sel de métaux alcalino-terreux pour le traitement des prurits et des dysesthésies oculaires ou palpébraux**
Verwendung von Erdalkalimetalsalzen in Mitteln zur Behandlung van Augen- oder Augenlidschmerzen oder Dysesthesien
Use of alkaline earth metal salts for the treatment of pruritis, eye or eyelid pain or disesthesies

(30) Priorité: 10.04.1995 FR 9504266
(43) Date de publication de la demande: 16.10.1996
(73) Titulaire: L'OREAL, 75008 Paris (FR)
(72) Inventeur: de Lacharriere, Olivier, F-75015 Paris (FR); Breton, Lionel, F-78000 Versailles (FR)
(74) Mandataire: Lhoste, Catherine

(56) Documents cités:
- EP-A- 0 451 300
- WO-A-93/18747
- WO-A-94/13305
- US-A- 5 328 701

## Description

La présente invention se rapporte à l'utilisation de sel de strontium pour la préparation d'une composition pharmaceutique pour traiter notamment par voie topique les prurits oculaires et/ou palpébraux et/ou les dysesthésies oculaires et/ou palpébrales. Elle se rapporte aussi à l'utilisation de sel de strontium dans une composition cosmétique destinée au soin ou au maquillage des yeux ou des paupières ainsi qu'à un procédé de maquillage des yeux sensibles.

Certains patients présentent très fréquemment sans qu'on n'en connaisse toujours la cause précise des sensations de démangeaisons ou prurit et des sensations dysesthésiques au niveau des yeux et des paupières. Il peut s'agir également de prurits ou sensations dysesthésies d'origine allergique.

Par sensations dysesthésiques, on entend des sensations de brûlures ou d'échauffement, de picotements, de fourmillements, d'inconforts et de tiraillements. Ces sensations peuvent s'associer à des rougeurs.

L'ensemble de ces signes ophtalmiques peuvent en outre s'associer à une rosacée et éventuellement à une conjonctivite.

Parmi les facteurs déclenchants les crises prurigineuses ou dysesthésiques ophtalmiques ou palpébrales, on peut citer les variations de température rapide, la chaleur et notamment l'exposition aux ultraviolets ou aux infrarouges, l'humidité relative basse, l'exposition aux vents violents ou aux courants d'air (soufflerie, air conditionné), l'application de tensioactifs (shampooing), l'exposition à des vapeurs toxiques ou irritantes (solvants) ou à des poussières, les gouttes ou topiques ophtalmologiques irritants, les topiques palpébraux dermatologiques ou cosmétiques irritants (alpha-hydroxyacides, rétinoïdes), ou l'utilisation de certains cosmétiques même lorsque ceux-ci ne sont pas connus comme particulièrement irritants. Comme autres facteurs déclenchants les crises prurigineuses ou dysesthésiques oculaires ou palpébrales, il faut, en outre, inclure les allergènes comme notamment le pollen, les poils d'animaux, les acariens, les moisissures.

Jusqu'alors, le mécanisme pathologique de ces signes était très mal connu et les dysesthésies oculaires et/ou palpébrales étaient traitées par des corticoïdes et également des antiseptiques locaux en pommade ophtalmique ou en gouttes.

Les corticoïdes sont relativement efficaces pour calmer les symptômes ci-dessus, mais malheureusement, ils présentent des effets secondaires souvent très pénalisants comme des atrophies. De plus, ils sensibilisent aux infections mycosiques ou bactériennes et leur cinétique d'action est souvent lente (plusieurs minutes à quelques heures). Par ailleurs, leur utilisation chronique peut conduire à une pharmacodépendance. En outre, leur action n'est pas immédiate et les sensations dysesthésiques subsistent pendant plusieurs minutes.

Il subsiste donc le besoin d'un traitement à effet immédiat des prurits et des dysesthésies oculaires et palpébraux ne présentant pas les inconvénients ci-dessus.

La présente invention a justement pour objet l'utilisation d'un ou plusieurs sels de strontium pour traiter ces affections, et notamment d'un ou plusieurs sels de strontium associés éventuellement à au moins un antagoniste de neuropeptide et/ou au moins un antagoniste de médiateur de l'inflammation.

La demanderesse a constaté que les dysesthésies oculaires et/ou palpébrales étaient liées à la stimulation des fibres nerveuses et au relargage par les terminaisons nerveuses sensitives de neuropeptides tels que par exemple la substance P et le CGRP (peptide dérivé du gène de la calcitonine : Calcitonin Gene Related Peptide en terminologie anglo-saxonne) ainsi que des médiateurs pro-inflammatoires libérés par suite de la fixation de certains neuropeptides (substance P notamment) sur les récepteurs mastocytaires. Ces médiateurs pro-inflammatoires sont plus spécialement l'histamine, l'héparine, la sérotonine, l'interleukine 1 (IL1), l'interleukine 6 (IL6), l'interleukine 8 (IL8) et le Tumor Necrosis Factor alpha (TNF-alpha). Un recrutement des globules blancs neutrophiles par certains neuropeptides (CGRP notamment) est également impliqué dans le processus inflammatoire.

Personne n'avait envisagé jusqu'à ce jour d'utiliser les sels de strontium pour traiter les prurits et/ou les dysesthésies oculaires ou palpébrales.

Aussi, la présente invention a pour objet l'utilisation d'au moins un sel de strontium pour la préparation d'une composition pharmaceutique ou dermatologique pour traiter les prurits oculaires ou palpébraux et/ou les dysesthésies oculaires ou palpébrales.

L'invention a encore pour objet une composition cosmétique, pharmaceutique ou dermatologique pour yeux sensibles contenant, dans un milieu cosmétiquement, pharmaceutiquement ou dermatologiquement acceptable, au moins un sel de strontium et au moins un antagoniste de neuropeptide et/ou au moins un antagoniste de médiateur de l'inflammation.

L'application de compositions contenant un ou plusieurs sels d'un ou plusieurs métaux alcalino-terreux associés notamment à un ou plusieurs antagonistes de neuropeptides et/ou antagoniste de médiateur de l'inflammation sur les yeux ou les paupières permet d'obtenir une nette diminution voire une disparition complète des sensations dysesthésiques et prurits oculaires ; on constate très rapidement, et en tout état de cause beaucoup plus rapidement qu'avec les corticoïdes, un effet calmant et apaisant, préventif et curatif sur les yeux et les paupières. En outre, on ne note aucune pharmacodépendance.

Grâce à ces sels de métaux alcalino-terreux, il en outre possible de concevoir des compositions cosmétiques pour yeux sensibles et en particulier des lotions démaquillantes ou de nettoyage des yeux, des produits de maquillage pour yeux sensibles et notamment des fards à paupières, des mascaras, des crayons ou des eye-liners pour yeux sensibles.

Aussi, l'invention à encore pour objet l'utilisation d'au moins un sel de strontium dans une composition cosmétique contenant un milieu cosmétiquement acceptable, destinée aux yeux sensibles.

L'invention a encore pour objet un procédé de maquillage des yeux sensibles et un procédé de traitement des yeux sensibles, consistant à appliquer sur les paupières, les cils ou sous les yeux sensibles une composition contenant au moins un sel de strontium dans un milieu cosmétiquement ou pharmaceutiquement acceptable.

La composition de l'invention contient un milieu cosmétiquement, pharmaceutiquement ou dermatologiquement acceptable, c'est-à-dire un milieu compatible avec la peau, et les yeux. La composition contenant le ou les sels de métal ou métaux alcalino-terreux est appliquée notamment par voie topique.

Comme sels de métaux alcalino-terreux utilisables dans l'invention, on peut citer les sels de baryum, de calcium, de magnésium, de strontium et/ou de béryllium. Ces sels peuvent être par exemple des carbonates, des bicarbonates, des sulfates, des glycérophosphates, des borates, des chlorures, des nitrates, des acétates, des hydroxydes, des persulfates ainsi que des sels d'α-hydroxyacides (citrates, tartrates, lactates, malates) ou d'acides de fruits, ou encore des sels d'acides aminés (aspartate, arginate, glycocholate, fumarate) ou des sels d'acides gras (palmitate, oléate, caséinate, béhénate). Par exemple, le sel est choisi parmi le nitrate de calcium ou de magnésium, le borate de calcium ou de magnésium, le chlorure de calcium ou de magnésium, le sulfate de calcium ou de magnésium et l'acétate de calcium ou de magnésium. Avantageusement, le sel est un sel de magnésium ou mieux de strontium et en particulier un chlorure ou un nitrate.

La demanderesse a trouvé de façon surprenante que l'aptitude des sels d'alcalino-terreux à traiter les yeux sensibles était dûe notamment au fait que ces sels étaient des inhibiteurs de la libération de TNF-alpha, voire même des inhibiteurs de la libération de neuropeptides comme le CGRP et la substance P.

Dans les compositions selon l'invention, les sels de métaux alcalino-terreux est utilisé de préférence en une quantité allant de 0,01 à 20 % du poids total de la composition, et en particulier en une quantité allant de 0,1 à 15 % du poids total de la composition et mieux de 0,5 % à 8 %.

Les compositions selon l'invention peuvent se présenter sous toutes les formes galéniques normalement utilisées pour une application topique ; la composition peut se présenter notamment sous forme de solutions aqueuses, hydroalcooliques ou huileuses, ou de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel aqueux ou anhydre, de microémulsions, de microcapsules, de microparticules, de dispersions vésiculaires de type ionique et/ou non ionique, de poudres compactées ou coulées. Ces compositions sont préparées selon les méthodes usuelles. Elles sont essentiellement destinées à une application topique.

Pour une application topique à visée thérapeutique, les compositions se présentent notamment sous forme de gel, crème, pommade pour le traitement des prurits et/ou dysesthésies palpébraux et sous forme de collyre ou de solution de lavage oculaire pour le traitement des prurits et/ou dysesthésies oculaires.

Pour une application cosmétique, les compositions peuvent notamment être constituées de crèmes de soin ou de protection pour yeux sensibles, de laits ou lotions de nettoyage ou de démaquillage des yeux sensibles, de produits de maquillage des yeux notamment sensibles comme des crayons, des mascaras, des eye-liners, des fards à paupières.

Les quantités des différents constituants des compositions selon l'invention sont celles classiquement utilisées dans les domaines considérés.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans les domaines cosmétique et dermatologique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la quantité d'huile peut aller jusqu'à plus de 90 % en poids du poids total de la composition.

De façon connue, la composition de l'invention peut contenir également des adjuvants habituels dans les domaines considérés, tels que les gélifiants hydrophiles ou lipophiles, les actifs hydrophiles ou lipophiles, les conservateurs, les antioxydants, les solvants, les parfums, les charges, les filtres solaires, les absorbeurs d'odeur, des pigments et les matières colorantes. Les quantités de ces différents adjuvants sont celles classiquement utilisées dans les domaines considérés, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclométhicone) et les huiles fluorées (perfluoropolyéthers). On peut aussi utiliser des alcools gras, des acides gras (acide stéarique) ou encore des cires (paraffine, carnauba, cire d'abeilles).

Comme émulsionnants utilisables dans l'invention, on peut citer par exemple le stéarate de glycérol, le polysorbate 60 ou 80 et le mélange de PEG-6/PEG-32/Glycol Stéarate vendu sous la dénomination de Tefose^{R} 63 par la société Gattefosse.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, les polyéthylènes et l'éthylcellulose.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon, les extraits bactériens ou végétaux, notamment d'Aloe Vera.

Comme actifs lipophiles, on peut utiliser le tocophérol (vitamine E) et ses dérivés, le rétinol (vitamine A) et ses dérivés, les acides gras essentiels, les céramides, les huiles essentielles.

On peut également associer les sels de métaux alcalino-terreux à des agents actifs, notamment des cicatrisants (par exemple vitamine B₁₂), des antiseptiques (par exemple acide borique), des antiallergiques (par exemple chromoglycate de sodium), des antiviraux (par exemple acyclovir), des anesthésiques (par exemple chlorhydrate de lidocaïne et dérivés), des anti-inflammatoires non stéroïdiens (par exemple indométhacine), des antagonistes de neuropeptides (antagonistes de substance P, de CGRP), et des antagonistes d'histamine, d'interleukine (IL1 notamment) ou de TNFα (Tumor Necrosis Factor-α) ainsi qu'une petite quantité de corticoïdes, ces agents actifs étant différents des sels de métaux d'alcalino-terreux.

La demanderesse définit un antagoniste de neuropeptide comme toute molécule d'origine organique ou minérale capable de produire une inhibition de la fixation réceptorielle du neuropeptide ou de produire une inhibition de la synthèse et/ou de la libération de ce neuropeptide par les fibres nerveuses sensitives.

De même, la demanderesse définit un antagoniste de médiateur de l'inflammation comme toute molécule d'origine organique ou minérale capable de produire une inhibition de la fixation réceptorielle de ce médiateur de l'inflammation ou de produire une inhibition de la synthèse et/ou de la libération de ce médiateur par les fibres nerveuses sensitives.

Avantageusement, les antagonistes de neuropeptides sont des antagonistes réceptoriels.

De préférence le ou les antagonistes de neuropeptides sont choisis parmi les antagonistes de substance P et les antagonistes de CGRP, notamment réceptoriels.

On peut utiliser dans l'invention par exemple comme antagoniste de CGRP, le CGRP 8-37, un anticorps anti-CGRP.

A titre d'exemple, les antagonistes de neuropeptide et les antagonistes de médiateur de l'inflammation peuvent être utilisés en une quantité représentant de 0,000001 à 10 % du poids total de la composition et mieux de 0,0001 à 5 %.

Comme antagonistes de médiateur de l'inflammation utilisables dans l'invention, on peut citer les dérivés de la diéthylène diamine telle que cinnarizine, cyclizine ; les dérivés de l'aminopropane (dexchlorophéniramine, tripolidine) ; des dérivés de phénothiazine (alimémazine, prométhazine) ; l'auranofine ; la lisophyline ; l'A802715 ; la sulfasalazine ; la cétirizine HCI ; la loratidine ; l'esbatine ; la sétastine HCI.

Des tests ont mis en évidence l'activité de différents sels de strontium comme antagonistes de substance P en référence au Spantide II, connu comme antagoniste de substance P, et comparativement à un autre sel de métal alcalino-terreux, le nitrate de calcium.

Le test a consisté à déterminer l'activité des différents composés sur la libération de substance P, provoquée par stimulation antidromique du nerf sciatique. Cette libération est visualisée par coloration avec un colorant (bleu Evans). La coloration est d'autant plus forte que la quantité de substance P libérée est importante. Autrement dit, plus l'antagoniste de substance P produit un effet inhibiteur sur cette libération, plus la coloration est faible.

Les résultats sont rassemblés dans les tableaux ci-dessous :

**Tableau 1**

| | Témoin véhicule | Spantide II 30 nmoles | Sr(NO₃)₂ 1 µmole | SrCl₂ 1 µmole | Ca(NO₃)₂ 1 µmole |
|---|---|---|---|---|---|
| µg/ml de bleu Evans | 8,19 ± 0,83 | 4,35 ± 0,46 | 4,84 ± 0,6 | 5,13 ± 0,9 | 11,25 ± 1,77 |
| % d'inhibition par rapport au témoin | - | 47 % | 41 % | 37 % | - |
| Statistiques | - | p<0,01 | p<0,05 | p<0,05 | non significatif |

Il ressort de ce tableau que les sels de strontium ont une activité importante d'inhibition de la libération de la substance P alors que le sel de calcium, à taux de concentration égale, n'a pas d'activité du tout.

**Tableau 2**

| | Témoin véhicule | Spantide II 30 nmoles | Sr(NO₃)₂ 3 µmole | SrCl₂ 1 µmole | Ca(NO₃)₂ 10 µmole |
|---|---|---|---|---|---|
| µg/ml de bleu Evans | 6,26 ± 1,09 | 3,63 ± 0,69 | 1,80 ± 0,68 | 2,71 ± 0,67 | 8,45 ± 1,55 |
| % d'inhibition par rapport au témoin | - | 42 % | 71 % | 57 % | - |
| Statistiques | - | p<0,05 | p<0,001 | p<0,01 | non significatif |

Il ressort de ce tableau que les sels de strontium ont une activité importante d'inhibition de la libération de la substance P alors que le sel de calcium n'a pas d'activité du tout, même à un taux de concentration plus important.

Le test suivant permet de montrer que les sels d'alcalino-terreux et plus spécialement le chlorure de magnésium, de strontium ou de calcium ainsi que le nitrate de strontium sont des inhibiteurs de TNF-alpha.

Ce test est réalisé sur des monocytes humains (lignée U937) stimulés par un ester de phorbol (PMA) selon la méthode décrite par Schindler et al (Correlations and interactions in the production of interleukin-6 (IL-6), IL-1, and Tumor Necrosis Factor (TNF) in human blood mononuclear cells : IL-6 suppresses IL-1 and TNF. Blood 75 : 40-47 (1990)).

L'ester de phorbol (PMA) stimule naturellement la synthèse et/ou la sécrétion de TNF-alpha par des monocytes humains en culture.

L'activité des molécules est évaluée en fonction de leurs aptitudes à diminuer, voire à supprimer cette sécrétion de TNF-alpha.

Les cellules (monocytes) sont incubées en présence de PMA à la concentration de 10 nM pendant 48 heures à 37 °C. Les quantités de TNF-alpha sécrétées sont quantifiées par dosage immuno-enzymatique (ECA) à l'aide de kits disponibles dans le commerce.

Chaque molécule est testée à 3 concentrations différentes (10⁻⁵, 10⁻⁴, et 10⁻³ M) et à chaque expérimentation une molécule de référence (dexaméthasone) est étudiée à 7 concentrations comme standard interne.

Les résultats sont exprimés en pourcentage d'inhibition par rapport au témoin positif (sans molécule à tester) après soustraction du bruit de fond. Un tableau synthétique regroupe les effets inhibiteurs moyennés, obtenus avec les composés. (Les résultats sont exprimés en % d'inhibition ; ils sont la moyenne de 3 mesures)

La valeur d'IC₅₀ (diminution de 50 % de la sécrétion, provoquée par le PMA) est calculée, pour la molécule de référence, à partir de la courbe de compétition selon un modèle de régression non linéaire.

**Tableau**

| test | composés | 10⁻⁵ M | 10⁻⁴ M | 10⁻³ M | référence | IC₅₀ |
|---|---|---|---|---|---|---|
| sécrétion de TNF-alpha induite par le PMA | SrCl₂7H2O | 14 | - | 54 | | |
| | M_{g}Cl₂ | - | 12 | 70 | | |
| | | | | | dexaméthasone | 4x10⁻⁹M |
| | CaCl₂, 7H₂O | 19 | 22 | 67 | | |
| | Sr(NO₃)₂ | - | 28 | 75 | | |

On constate que l'inhibition de TNF-alpha augmente avec la concentration en sel.

Les exemples suivants illustrent l'invention. Dans ces exemples, les proportions indiquées sont des pourcentages en poids.

### Exemple 1 : Collyre

| | |
|---|---|
| Chlorure de strontium | 3 % |
| Excipient : | qsp 100% |
| Chlorure de sodium | |
| Borate de sodium | |
| Polysorbate 80 | |
| Acide borique | |
| Eau | |

### Exemple 2 : Pommade

| | |
|---|---|
| Nitrate de magnésium | 5 % |
| Excipient : | qsp 100% |
| Chlorure de benzalkonium | |
| Edetate de sodium | |
| D-mannitol | |
| Carbomer | |
| Soude | |
| Eau | |

### Exemple 3 : Solution

| | |
|---|---|
| Borate de calcium | 2 % |
| Excipient : | |
| Acide borique | 5 % |
| Chlorure de sodium | 0,3 % |
| Borate de phénylmercure | 0,5 % |
| Eau | qsp 100 % |

### Exemples 4 et 5: Pommades

Ces exemples se distinguent de l'exemple 2 par l'ajout de 0,1 % de sendide respectivement loratidine.

### Exemple 6

Cet exemple se différencie de l'exemple 1 par l'emploi de 5% de nitrate de strontium.

## Revendications

1. Utilisation d'au moins un sel de strontium pour la préparation d'une composition pharmaceutique ou dermatologique pour traiter les prurits oculaires ou palpébraux et/ou les dysesthésies oculaires ou palpébrales.

2. Utilisation d'au moins un sel de strontium dans une composition cosmétique contenant un milieu cosmétiquement acceptable, destinée aux yeux sensibles.

3. Utilisation selon la revendication 1 ou 2, caractérisée en ce que le sel de strontium est choisi parmi les chlorures, carbonates, borates, nitrates, acétates, hydroxydes, sulfates, acides de fruits et acides aminés de strontium.

4. Utilisation selon l'une quelconque des revendications précédentes, caractérisée en ce que le sel est choisi parmi le chlorure et le nitrate de strontium.

5. Utilisation selon l'une quelconque des revendications précédentes, caractérisée en ce que le sel de strontium est utilisé en une quantité allant de 0,01 à 20 % en poids par rapport au poids total de la composition.

6. Utilisation selon l'une quelconque des revendications précédentes, caractérisée en ce que le sel de strontium est utilisé en une quantité allant de 0,5 à 8 % en poids par rapport au poids total de la composition.

7. Utilisation selon l'une quelconque des revendications précédentes, caractérisée en ce que la composition contient en outre au moins un actif choisi parmi les protéines ou les hydrolysats de protéine, les acides aminés, les polyols, l'urée, les sucres et les dérivés de sucre, les vitamines, l'amidon, les extraits végétaux, les céramides, les huiles essentielles, les filtres solaires.

8. Utilisation selon l'une quelconque des revendications précédentes, caractérisée en ce que la composition contient en outre au moins un agent choisi parmi les agents cicatrisants, antiseptiques, antiallergiques, anesthésiques, antiviraux, anti-inflammatoires non stéroïdiens, les antagonistes de neuropeptides et les antagonistes de médiateur de l'inflammation, autres que le sel.

9. Utilisation selon l'une quelconque des revendications précédentes, caractérisée en ce que la composition contient en outre au moins un agent choisi parmi les antagonistes de substance P et les antagonistes de CGRP.

10. Utilisation selon la revendication 8 ou 9, caractérisée en ce que l'antagoniste de neuropeptide est un antagoniste réceptoriel.

11. Utilisation selon l'une quelconque des revendications précédentes, caractérisée en ce que la composition se présente sous une forme appropriée à une application topique.

12. Utilisation selon l'une quelconque des revendications précédentes, caractérisée en ce que la composition est une solution aqueuse, huileuse ou hydroalcoolique, une émulsion eau-dans-huile, une émulsion huile-dans-eau, une microémulsion, un gel aqueux, un gel anhydre, un sérum, une dispersion de vésicules, de microcapsules ou de microparticules, une poudre compactée ou coulée.

13. Utilisation selon l'une quelconque des revendications précédentes, caractérisée en ce que la composition est sous forme de gel, de crème ou de pommade pour le traitement des prurits et/ou dysesthésies palpébraux.

14. Utilisation selon l'une quelconque des revendications précédentes, caractérisée en ce que la composition est sous forme de collyre pour le traitement des prurits et/ou dysesthésies oculaires.

15. Utilisation selon l'une quelconque des revendications 1 à 13 caractérisée en ce que la composition est une composition de lait ou lotion de nettoyage ou démaquillage des yeux sensibles, un produit de maquillage des yeux sensibles tel qu'un crayon, mascara, eye-liner, fard à paupières.

16. Procédé de maquillage des yeux sensibles, consistant à appliquer sur les paupières, les cils ou sous les yeux des personnes à yeux sensibles une composition cosmétique contenant au moins un sel de strontium dans un milieu cosmétiquement acceptable.

17. Composition cosmétique, pharmaceutique ou dermatologique pour yeux sensibles, contenant, dans un milieu cosmétiquement, pharmaceutiquement ou dermatologiquement acceptable, au moins un sel de strontium et au moins un antagoniste de neuropeptide et/ou au moins un antagoniste de médiateur de l'inflammation.

18. Composition selon la revendication 17, caractérisée en ce que le sel de strontium est choisi parmi les chlorures, carbonates, borates, nitrates, acétates, hydroxydes, sulfates, les sels d'acides de fruits et d'acides aminés.

19. Composition selon l'une des revendications 17 ou 18, caractérisée en ce que le sel est le chlorure ou le nitrate de strontium.

20. Composition selon l'une quelconque des revendications 17 à 19, caractérisée en ce que le sel de strontium est utilisé en une quantité allant de 0,01 à 20% en poids par rapport au poids total de la composition.

21. Composition selon l'une quelconque des revendications 17 à 20, caractérisée en ce que le sel de strontium est utilisé en une quantité allant de 0,5 à 8% en poids par rapport au poids total de la composition.

22. Composition selon l'une quelconque des revendications 17 à 21, caractérisée en ce que la composition contient en outre au moins un actif choisi parmi les protéines ou les hydrolysats de protéine, les acides aminés, les polyols, l'urée, les sucres et les dérivés de sucre, les vitamines, l'amidon, les extraits végétaux, les céramides, les huiles essentielles, les filtres solaires.

23. Composition selon l'une quelconque des revendications 17 à 22, caractérisée en ce que la composition contient en outre au moins un agent choisi parmi les agents cicatrisants, antiseptiques, antiallergiques, anesthésiques, antiviraux, anti-inflammatoires non stéroïdiens.

24. Composition selon l'une quelconque des revendications 17 à 23, caractérisée en ce que l'antagoniste de neuropeptide est choisi parmi les antagonistes de substance P et les antagonistes de CGRP.

25. Composition selon l'une quelconque des revendications 17 à 24, caractérisée en ce que l'antagoniste de neuropeptide est un antagoniste réceptoriel.

26. Composition selon l'une quelconque des revendications 17 à 25, caractérisée en ce que la composition se présente sous une forme appropriée à une application topique, injectable ou ingérable.

27. Composition selon l'une quelconque des revendications 17 à 26, caractérisée en ce que la composition est une solution aqueuse, huileuse ou hydroalcoolique, une émulsion eau-dans-huile, une émulsion huile-dans-eau, une microémulsion, un gel aqueux, un gel anhydre, un sérum, une dispersion de vésicules, de microcapsules ou de microparticules, une poudre compactée ou coulée.

28. Composition selon l'une quelconque des revendications 17 à 27, caractérisée en ce que la composition se présente sous forme appropriée à une application topique.

29. Composition selon l'une quelconque des revendications 17 à 28, caractérisée en ce qu'elle se présente sous forme d'un lait ou lotion de nettoyage ou démaquillage des yeux sensibles, un produit de maquillage des yeux sensibles tel qu'un crayon, mascara, eye-liner, fard à paupières.

## Patentansprüche

1. Verwendung mindestens eines Strontiumsalzes zur Herstellung einer pharmazeutischen oder dermatologischen Zusammensetzung zur Behandlung von okularem oder palpebralen Juckreiz und/oder okularer oder palpebraler Dysästhesie.

2. Verwendung mindestens eines Strontiumsalzes in einer kosmetischen Zusammensetzung, die ein kosmetisch akzeptables Medium enthält und für empfindliche Augen vorgesehen ist.

3. Verwendung nach Anspruch 1 oder 2, dadurch gekennzeichnet, daß das Strontiumsalz unter den Chloriden, Carbonaten, Boraten, Nitraten, Acetaten, Hydroxiden, Sulfaten, Fruchtsäuresalzen und Aminosäuresalzen von Strontium ausgewählt ist.

4. Verwendung nach einem der vorhergehenden Ansprüche, dadurch gekennzeichnet, daß das Salz unter Strontiumchlorid und Strontiumnitrat ausgewählt ist.

5. Verwendung nach einem der vorhergehenden Ansprüche, dadurch gekennzeichnet, daß das Strontiumsalz in einem Mengenanteil von 0,01 bis 20 Gew.-%, bezogen auf das Gesamtgewicht der Zusammensetzung, verwendet wird.

6. Verwendung nach einem der vorhergehenden Ansprüche, dadurch gekennzeichnet, daß das Strontiumsalz in einem Mengenanteil von 0,5 bis 8 Gew.-%, bezogen auf das Gesamtgewicht der Zusammensetzung, verwendet wird.

7. Verwendung nach einem der vorhergehenden Ansprüche, dadurch gekennzeichnet, daß die Zusammensetzung ferner einen Wirkstoff enthält, der unter den Proteinen oder Proteinhydrolysaten, Aminosäuren, Polyolen, Harnstoff, Zuckern und Zucker-derivaten, Vitaminen, Stärke, Pflanzenextrakten, Ceramiden, etherischen Ölen und Sonnenschutzfiltern ausgewählt ist.

8. Verwendung nach einem der vorhergehenden Ansprüche, dadurch gekennzeichnet, daß die Zusammensetzung ferner mindestens ein Mittel enthält, das unter den Vernarbungsmitteln, antiseptischen Mitteln, antiallergischen Mitteln, anästhesierenden Mitteln, antiviralen Mitteln, nicht steroidalen entzündungshemmenden Mitteln, Neuropeptid-Antagonisten und Antagonisten von Entzündungsmediatoren, die von dem Salz verschieden sind, ausgewählt ist.

9. Verwendung nach einem der vorhergehenden Ansprüche, dadurch gekennzeichnet, daß die Zusammensetzung ferner mindestens ein Mittel enthält, das unter den Substanz P-Antagonisten und den CGRP-Antagonisten ausgewählt ist.

10. Verwendung nach Anspruch 8 oder 9, dadurch gekennzeichnet, daß der Neuropeptid-Antagonist ein Rezeptor-Antagonist ist.

11. Verwendung nach einem der vorhergehenden Ansprüche, dadurch gekennzeichnet, daß die Zusammensetzung in einer zur topischen Anwendung geeigneten Form vorliegt.

12. Verwendung nach einem der vorhergehenden Ansprüche, dadurch gekennzeichnet, daß die Zusammensetzung eine wässrige, ölige oder wässrig-alkoholische Lösung, Wasser-in-Öl-Emulsion, Öl-in-Wasser-Emulsion, Mikroemulsion, ein wässriges Gel, wasserfreies Gel, Serum, eine Vesikeldispersion, Mikrokapseldispersion, Mikropartikeldispersion oder ein gepreßtes oder gegossenes Pulver ist.

13. Verwendung nach einem der vorhergehenden Ansprüche, dadurch gekennzeichnet, daß die Zusammensetzung in Form eines Gels, einer Creme oder einer Salbe zur Behandlung von palpebralem Juckreiz und/oder palpebralen Dysästhesien vorliegt.

14. Verwendung nach einem der vorhergehenden Ansprüche, dadurch gekennzeichnet, daß die Zusammensetzung in Form von Augentropfen zur Behandlung von okularem Juckreiz und/oder okularen Dysästhesien vorliegt.

15. Verwendung nach einem der Ansprüche 1 bis 13, dadurch gekennzeichnet, daß die Zusammensetzung eine Milch oder Lotion zur Reinigung oder zum Abschminken von empfindlichen Augen oder ein Schminkprodukt für empfindliche Augen, beispielsweise ein Konturenstift, Mascara, Eyeliner oder Lidschatten, ist.

16. Verfahren zum Schminken empfindlicher Augen, das darin besteht, bei Personen mit empfindlichen Augen auf die Lider, die Wimpern oder unter die Augen eine kosmetische Zusammensetzung aufzubringen, die in einem kosmetisch akzeptablen Medium ein Strontiumsalz enthält.

17. Kosmetische, pharmazeutische oder dermatologische Zusammensetzung für empfindliche Augen, die in einem kosmetisch, pharmazeutisch oder dermatologisch akzeptablen Medium mindestens ein Strontiumsalz und mindestens einen Neuropeptid-Antagonisten und/oder mindestens einen Antagonisten eines Entzündungsmediators enthält.

18. Zusammensetzung nach Anspruch 17, dadurch gekennzeichnet, daß das Strontiumsalz unter den Chloriden, Carbonaten, Boraten, Nitraten, Acetaten, Hydroxiden, Sulfaten, Fruchtsäuresalzen und Aminosäuresalzen ausgewählt ist.

19. Zusammensetzung nach einem der Ansprüche 17 oder 18, dadurch gekennzeichnet, daß das Salz das Strontiumchlorid und Strontiumnitrat ist.

20. Zusammensetzung nach einem der Ansprüche 17 bis 19, dadurch gekennzeichnet, daß das Strontiumsalz in einem Mengenanteil von 0,5 bis 8 Gew.-%, bezogen auf das Gesamtgewicht der Zusammensetzung, verwendet wird.

21. Zusammensetzung nach einem der Ansprüche 17 bis 20, dadurch gekennzeichnet, daß das Strontiumsalz in einem Mengenanteil von 0,5 bis 8 Gew.-%, bezogen auf das Gesamtgewicht der Zusammensetzung, verwendet wird.

22. Zusammensetzung nach einem der Ansprüche 17 bis 21, dadurch gekennzeichnet, daß die Zusammensetzung ferner einen Wirkstoff enthält, der unter den Proteinen oder Proteinhydrolysaten, Aminosäuren, Polyolen, Harnstoff, Zuckern und Zucker-derivaten, Vitaminen, Stärke, Pflanzenextrakten, Ceramiden, etherischen Ölen und Sonnenschutzfiltern ausgewählt ist.

23. Zusammensetzung nach einem der Ansprüche 17 bis 22, dadurch gekennzeichnet, daß die Zusammensetzung ferner mindestens ein Mittel enthält, das unter den Vernarbungsmitteln, antiseptischen Mitteln, antiallergischen Mitteln, anästhesierenden Mitteln, antiviralen Mitteln und nicht steroidalen entzündungshemmenden Mitteln ausgewählt ist.

24. Zusammensetzung nach einem der Ansprüche 17 bis 23, dadurch gekennzeichnet, daß der Neuropeptid-Antagonist unter den Substanz P-Antagonisten und den CGRP-Antagonisten ausgewählt ist.

25. Zusammensetzung nach einem der Ansprüche 17 bis 24, dadurch gekennzeichnet, daß der Neuropeptid-Antagonist ein Rezeptor-Antagonist ist.

26. Zusammensetzung nach einem der Ansprüche 17 bis 25, dadurch gekennzeichnet, daß die Zusammensetzung in einer für eine topische Anwendung, zum Injizieren oder Einnehmen geeigneten Form vorliegt.

27. Zusammensetzung nach einem der Ansprüche 17 bis 26, dadurch gekennzeichnet, daß die Zusammensetzung eine wäßrige, ölige oder wässrig-alkoholische Lösung, Wasser-in-Öl-Emulsion, Öl-in-Wasser-Emulsion, Mikroemulsion, ein wässriges Gel, wasserfreies Gel, Serum, eine Vesikeldispersion, Mikrokapseldispersion, Mikropartikeldispersion oder ein verpresstes oder gegossenes Pulver ist.

28. Zusammensetzung nach einem der Ansprüche 17 bis 27, dadurch gekennzeichnet, daß sie in einer zur topischen Anwendung geeigneten Form vorliegt.

29. Zusammensetzung nach einem der Ansprüche 17 bis 28, dadurch gekennzeichnet, daß sie als Milch oder Lotion zur Reinigung oder zum Abschminken von empfindlichen Augen oder Schminkprodukt für empfindliche Augen, beispielsweise Konturenstift, Mascara, Eyeliner oder Lidschatten, vorliegt.

## Claims

1. Use of at least one strontium salt for the preparation of a pharmaceutical or dermatological composition for treating ocular or palpebral pruritus and/or ocular or palpebral dysesthesia.

2. Use of at least one strontium salt in a cosmetic composition containing a cosmetically acceptable medium, which is intended for sensitive eyes.

3. Use according to Claim 1 or 2, characterized in that the strontium salt is chosen from the chlorides, carbonates, borates, nitrates, acetates, hydroxides, sulphates, fruit acids and amino acids of strontium.

4. Use according to any one of the preceding claims, characterized in that the salt is chosen from strontium chloride and strontium nitrate.

5. Use according to any one of the preceding claims, characterized in that the strontium salt is used in an amount ranging from 0.01% to 20% by weight relative to the total weight of the composition.

6. Use according to any one of the preceding claims, characterized in that the strontium salt is used in an amount ranging from 0.5% to 8% by weight relative to the total weight of the composition.

7. Use according to any one of the preceding claims, characterized in that the composition also contains at least one active agent chosen from proteins and protein hydrolysates, amino acids, polyols, urea, sugars and sugar derivatives, vitamins, starch, plant extracts, ceramides, essential oils and sunscreens.

8. Use according to any one of the preceding claims, characterized in that the composition also contains at least one agent chosen from cicatrizing agents, antiseptic agents, antiallergic agents, anaesthetics, antiviral agents, anti-inflammatory agents, non-steroidal agents, neuropeptide antagonists and inflammation-mediator antagonists, other than the salt.

9. Use according to any one of the preceding claims, characterized in that the composition also contains at least one agent chosen from substance P antagonists and CGRP antagonists.

10. Use according to Claim 8 or 9, characterized in that the neuropeptide antagonist is a receptor antagonist.

11. Use according to any one of the preceding claims, characterized in that the composition is in a form which is suitable for topical application.

12. Use according to any one of the preceding claims, characterized in that the composition is an aqueous, oily or aqueous-alcoholic solution, a water-in-oil emulsion, an oil-in-water emulsion, a microemulsion, an aqueous gel, an anhydrous gel, a serum, a dispersion of vesicles, microcapsules or microparticles, or a compacted or cast powder.

13. Use according to any one of the preceding claims, characterized in that the composition is in the form of gel, cream or ointment for the treatment of pruritus and/or palpebral dysesthesia.

14. Use according to any one of the preceding claims, characterized in that the composition is in the form of eye drops for the treatment of pruritus and/or ocular dysesthesia.

15. Use according to any one of Claims 1 to 13, characterized in that the composition is a cleansing or make-up-removing milk or lotion composition for sensitive eyes, or a make-up product for sensitive eyes, such as a crayon, mascara, eyeliner or eye shadow.

16. Process for making up sensitive eyes, which consists in applying to the eyelids, the eyelashes or under the eyes of individuals with sensitive eyes a cosmetic composition containing at least one strontium salt in a cosmetically acceptable medium.

17. Cosmetic, pharmaceutical or dermatological composition for sensitive eyes, containing, in a cosmetically, pharmaceutically or dermatologically acceptable medium, at least one strontium salt and at least one neuropeptide antagonist and/or at least one inflammation-mediator antagonist.

18. Composition according to Claim 17, characterized in that the strontium salt is chosen from the chlorides, carbonates, borates, nitrates, acetates, hydroxides, sulphates, fruit acid salts and amino acid salts.

19. Composition according to either of Claims 17 and 18, characterized in that the salt is strontium chloride or strontium nitrate.

20. Composition according to any one of Claims 17 to 19, characterized in that the strontium salt is used in an amount ranging from 0.5% to 8% by weight relative to the total weight of the composition.

21. Composition according to any one of Claims 17 to 20, characterized in that the strontium salt is used in an amount ranging from 0.5% to 8% by weight relative to the total weight of the composition.

22. Composition according to any one of Claims 17 to 21, characterized in that the composition also contains at least one active agent chosen from proteins and protein hydrolysates, amino acids, polyols, urea, sugars and sugar derivatives, vitamins, starch, plant extracts, ceramides, essential oils and sunscreens.

23. Composition according to any one of Claims 17 to 22, characterized in that the composition also contains at least one agent chosen from cicatrizing agents, antiseptic agents, antiallergic agents, anaesthetics, antiviral agents and non-steroidal anti-inflammatory agents.

24. Composition according to any one of Claims 17 to 23, characterized in that the neuropeptide antagonist is chosen from substance P antagonists and CGRP antagonists.

25. Composition according to any one of Claims 17 to 24, characterized in that the neuropeptide antagonist is a receptor antagonist.

26. Composition according to any one of Claims 17 to 25, characterized in that the composition is in a form which is suitable for topical, injectable or ingestible application.

27. Composition according to any one of Claims 17 to 26, characterized in that the composition is an aqueous, oily or aqueous-alcoholic solution, a water-in-oil emulsion, an oil-in-water emulsion, a microemulsion, an aqueous gel, an anhydrous gel, a serum, a dispersion of vesicles, of microcapsules or of microparticles, or a compacted or cast powder.

28. Composition according to any one of Claims 17 to 27, characterized in that the composition is in a form which is suitable for topical application.

29. Composition according to any one of Claims 17 to 28, characterized in that it is in the form of a cleansing or make-up-removing milk or lotion for sensitive eyes, or a make-up product for sensitive eyes such as a crayon, mascara, eyeliner or eye shadow.
